# EUROPEAN PATENT APPLICATION

(11) **EP 4 600 926 A1**
(43) Date of publication of application: **13.08.2025**
(21) Application number: 25155715.3
(22) Date of filing: 04.02.2025
(51) Int. Cl.: G08B 21/04, G08B 25/00, A61B 5/00

(54) **APPARATUS, METHODS AND COMPUTER PROGRAMS FOR DETECTING CRITICAL EVENTS**

(30) Priority: 12.02.2024 GB 202401915
(71) Applicant: Nokia Technologies Oy, 02610 Espoo (FI)
(72) Inventor: THANGARAJAN, Ashok Samraj, Cambridge (GB); MONTANARI, Alessandro, Cambridge (GB); AL-NAIMI, Khaldoon, Beaconsfield (GB); FERLINI, Andrea, Cambridge (GB); DANG, Ting, Cambridge (GB); LIU, Yang, Cambridge (GB)
(74) Representative: Nokia EPO representatives

(57) **Abstract**

Examples of the disclosure relate to apparatus, methods and computer programs for detecting critical events. In examples of the disclosure an apparatus determines that a prospective critical event has been detected and requests data from one or more local devices wherein the data is collected in accordance with instructions previously provided by the apparatus. The apparatus receives the data from the one or more local devices and uses the received data to confirm if the prospective critical event is a critical event.

## Description

### TECHNOLOGICAL FIELD

Examples of the disclosure relate to apparatus, methods and computer programs for detecting critical events. Some relate to apparatus, methods and computer programs for enabling confirmation that a detected event is a critical event.

### BACKGROUND

Electronic devices comprising one or more sensors can be used to detect the occurrence of the events. The events can relate to a person or other subject. For example, heart rate monitors can monitor a user's heart rate and detect cardiac events or a motion sensor such as an IMU can detect falls or other impacts.

### BRIEF SUMMARY

According to various, but not necessarily all, examples of the disclosure there is provided an apparatus comprising means for:
determining that a prospective critical event has been detected;
requesting data from one or more local devices wherein the data is collected in accordance with instructions previously provided by the apparatus;
receiving the data from the one or more local devices; and
using the received data to confirm if the prospective critical event is a critical event.

The means may be for detecting the prospective critical event.

The means may be for receiving a notification that the prospective critical event has been detected by at least one of the one or more local devices.

The request for data may be transmitted automatically.

Using the received data to confirm if the prospective critical event is a critical event may comprise analyzing a correlation between the received data and expected data for the critical event.

The means may be for providing an output based, at least in part, on whether a critical event is confirmed.

The output may comprise at least one of;
an alert to a user of the apparatus;
an instruction to one or more local devices to provide an alert; or
a communication to an assistance provider.

According to various, but not necessarily all, examples of the disclosure there is provided a method comprising:
determining that a prospective critical event has been detected;
requesting data from one or more local devices wherein the data is collected in accordance with instructions previously provided by the apparatus;
receiving the data from the one or more local devices; and
using the received data to confirm if the prospective critical event is a critical event.

According to various, but not necessarily all, examples of the disclosure there is provided a computer program comprising instructions which, when executed by an apparatus, cause the apparatus to perform at least:
determining that a prospective critical event has been detected;
requesting data from one or more local devices wherein the data is collected in accordance with instructions previously provided by the apparatus;
receiving the data from the one or more local devices; and
using the received data to confirm if the prospective critical event is a critical event.

According to various, but not necessarily all, examples of the disclosure there is provided an apparatus comprising means for:
identifying at least one event that is detectable by a first device as a critical event;
determining data for confirming that a potential critical event detected by the first device is a critical event;
determining one or more local devices capable of providing the data; and
transmitting instructions to the one or more local devices wherein the instructions indicate the data that is to be recorded by the respective local devices.

The events that are detectable by the first device may be determined, at least in part, by one or more sensors of the first device.

The first device may comprise at least one of:
a heart rate sensor;
a photoplethysmography sensor;
a temperature sensor;
a motion sensor;
a pressure sensor;
an inertial measurement unit; or
a microphone.

The critical event may relate to a health condition of a user of the first device.

Identifying a critical event may comprises accessing a database of detectable events and determining if an event is indicated as critical in the database.

Determining data for confirming that a potential critical event detected by the first device is a critical event may comprise accessing metadata associated with the critical event.

The instructions transmitted to the respective local devices may comprise at least one of:
a type of data that is to be recorded; or
a time interval for which the data is to be recorded.

The instructions may be transmitted during a set up procedure of at least one of the first device and the one or more local devices.

At least one of the first device and the one or more local devices may be a wearable device.

At least one of the first device and the one or more local devices may be configured to monitor one or more biological parameters of the user.

The apparatus may be comprised within the first device.

The apparatus may be configured to communicate with the first device.

According to various, but not necessarily all, examples of the disclosure there is provided a method comprising:
identifying at least one event that is detectable by a first device as a critical event;
determining data for confirming that a potential critical event detected by the first device is a critical event;
determining one or more local devices capable of providing the data; and
transmitting instructions to the one or more local devices wherein the instructions indicate the data that is to be recorded by the respective local devices.

According to various, but not necessarily all, examples of the disclosure there is provided a computer program comprising instructions which, when executed by an apparatus, cause the apparatus to perform at least:
identifying at least one event that is detectable by a first device as a critical event;
determining data for confirming that a potential critical event detected by the first device is a critical event;
determining one or more local devices capable of providing the data; and
transmitting instructions to the one or more local devices wherein the instructions indicate the data that is to be recorded by the respective local devices.

While the above examples of the disclosure and optional features are described separately, it is to be understood that their provision in all possible combinations and permutations is contained within the disclosure. It is to be understood that various examples of the disclosure can comprise any or all the features described in respect of other examples of the disclosure, and vice versa. Also, it is to be appreciated that any one or more or all the features, in any combination, may be implemented by/comprised in/performable by an apparatus, a method, and/or computer program instructions as desired, and as appropriate. The description of a function should additionally be considered to also disclose any means suitable for performing that function

### BRIEF DESCRIPTION

Some examples will now be described with reference to the accompanying drawings in which:
FIG. 1 shows an example system
FIG. 2 shows an example method;
FIG. 3 shows an example method;
FIG. 4 shows an example control flow;
FIG. 5 shows an example control flow;
FIG. 6 shows an example software architecture;
FIG. 7 shows an example system; and
FIG. 8 shows an example controller.

The figures are not necessarily to scale. Certain features and views of the figures can be shown schematically or exaggerated in scale in the interest of clarity and conciseness. For example, the dimensions of some elements in the figures can be exaggerated relative to other elements to aid explication. Corresponding reference numerals are used in the figures to designate corresponding features. For clarity, all reference numerals are not necessarily displayed in all figures.

### DETAILED DESCRIPTION

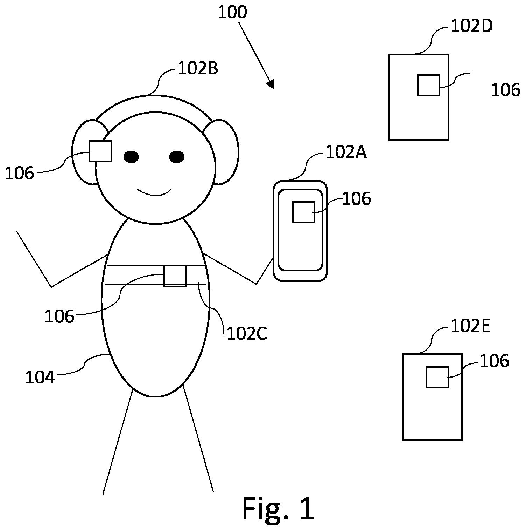

Fig. 1 shows an example system 100 that can be used to implement examples of the disclosure. The system 100 comprises multiple devices 102. In this example five devices 102 are shown. The system 100 can comprise any number of multiple devices 102 in examples of the disclosure.

The devices 102 that are comprised within the system 100 can comprise any suitable type of devices 102. The devices 102 within the system 100 can be configured to detect events. At least some of the respective devices 102 can comprise sensors that enable one or more events to be detected. The types of sensors that are used can depend upon the type of devices 102 within the system 100 and/or the types of events that are to be detected and/or any other suitable factors.

The example system of Fig. 1 comprises five devices 102. Other numbers of devices could be used in other examples. The devices 102 in the system 100 are local to each other. That is the devices 102 can communicate via short range wireless communication protocols or any other suitable means. The devices 102 could be within the same room or within several meters of each other.

In the example system 100 of Fig. 1 one of the devices 102A comprises a user electronics device. The user electronics device can be a communications device such as a smart phone. The user electronics device is a device that is used by or is otherwise associated with the user 104.The user electronics device can be a device that a user 104 usually has near to them. For example, it can be a device that is often in their pockets or kept close to the user 104 even if not currently in active use.

In the example of Fig. 1 the system 100 also comprises some wearable devices 102B, 102C. The wearable devices 102B, 102C can be devices that are worn by the user 104. These are devices that can be attached to the user's body. In some examples, one or more of the wearable devices 102B, 102C can be configured to monitor physiological parameters of the user 104. In such cases, one or more of the wearable devices 102B, 102C can comprise one or more sensors that can be used to monitor physiological parameters of the user 104. In some examples, one or more of the wearable devices 102B, 102C can be configured to provide outputs for the user 104. In such cases, one or more of the wearable devices 102B, 102C can comprise one or more output devices such as a loudspeaker or display or any other suitable means.

In this example, a first wearable device 102B comprises a head set. The head set is configured to be worn on the user's head. The head set can be configured to provide audio outputs to the user 104. The head set can comprise loudspeakers configured to provide audio outputs to the user 104. When the head set is worn on the user's head the loudspeakers are positioned close to the user's ears.

In some examples the head set can also comprise one or more sensors. For instance, the head set could comprise an inertial measurement unit (IMU) that can be used to detect the position or movements of the user's head. The information obtained by such sensors can be used for providing spatial audio to the user 104 but can also be used for detecting events relating to the user 104 such as a fall or other impact. In some examples a head set could comprise other sensors such as photoplethysmography sensors that can measure a user's heart rate or blood flow.

The head set can therefore perform multiple functions in that it can provide audio outputs to a user and also can use one or more sensors to collect data relating to events. Other types of wearable devices can also be used to perform multiple functions. For instance, other types of output devices could be used such as ear buds, smart glasses, virtual reality head sets or any other suitable devices. Other types of wearable devices could be used for other functions. For instance, a smart watch or smart ring could be worn on a user's wrist or finger and could be used for communications or for any other suitable use but could also be configured to monitor a user's movements, heart rate, and/or any other suitable physiological parameter.

In the example of Fig. 1 a second wearable device 102C comprises a heart rate monitor. The heart rate monitor comprises one or more sensors that are configured to measure a user's heart rate and/or any other physiological characteristics. In the example of Fig. 1 the heart rate monitor 102C is configured to be worn around the user's torso. Other heart types of heart rate monitor can be worn in other locations on the user's body.

The heart rate monitor can be configured for the primary purpose of monitoring heart rate and other related physiological parameters. Other wearable devices that are primarily for monitoring physiological parameters could be used in other examples. For instance, a wearable device could be configured to monitor heart rate or blood sugar levels or any other suitable parameter.

The system 100 of Fig. 1 also comprises other local devices 102D, 102E. The other local devices 102D, 102E can be in the same area as the user 104 but might not be associated specifically with the user 104. The other local devices 102D, 102E could comprise devices that could provide functions or outputs for multiple users. The other local devices 102D, 102E could be configured to monitor an area such as a room rather than a specific user 104. For example, the other local devices 102D, 102E could comprise smart speakers that could comprise microphones that can detect sound from the user 104 or any other objects in the local area. Other types of local devices 102D, 102E could comprise cameras, motion sensors, internet of things devices or other devices used for monitoring the area around the user 104.

The devices 102 in the system 100 can be configured to collect data that can be used to detect events. The respective devices 102 in the system 100 can comprise one or more sensors 106 that can be configured to be collect the data. Different devices 102 can comprise different types of sensors 106. The different types of sensors 106 can detect different parameters.

The sensors 106 that are used can depend upon the type of device 102. For example, a wearable device can comprise sensors 106 that can be configured to detect physiological parameters of the user 104. These sensors 106 could comprise heart rate sensors, photoplethysmography sensors, temperature sensors, motion sensors, inertial measurement units, or any other suitable types of sensors. Devices 102 that are configured to monitor the environment of the user could comprise cameras, microphones, motion sensors and/or any other suitable type of sensor 106.

The devices 102 within the system can be configured to communicate with each other. The respective devices 102 can comprise transceivers that enable the devices 102 to communicate with each other. The devices 102 can communicate using wireless communications protocols or any other suitable means.

In examples of the disclosure one or more of the devices 102 can be configured to detect a critical event. A critical event can be an event that has been flagged as a critical event. The critical events can be flagged by a task manager or any other suitable means. The task manager that flags an event as critical can be implemented in one of the devices 102. For example, the task manager could be implemented in a user electronics device such as a smart phone, or any other suitable type of device.

In some examples the critical events can be user definable. For example, a user of the device that implements the task manager can use the task manager to select one or more events that are to defined as critical.

The events that are classed as critical can be dependent upon the implementation of the system. The critical events can comprise a subset of all of the events that can be detected by the devices 102 in the system 100. The critical events can be distinguished from the other events so that different outputs or type of outputs can be provided in response to a critical events.

A critical event can be an event that happens to the user 104. Where the system 100 is configured to monitor biological parameters of a user 104 an event can be classed as critical if the event could be considered to be, dangerous or cause the user 104 to require assistance from a medical professional or other source of help. Examples of critical events could be cardiac events, drops in blood sugar levels, a fall or other impact, or other suitable events.

The critical event can relate to a health condition of a user of the first device. The critical event can relate to a health condition which means that the user requires assistance or needs to perform some action to avoid or address an illness or injury.

If a critical event occurs then one or more of the devices 102 in the system 100 can provide an appropriate output. The appropriate output could comprise raising an alarm, contacting assistance or any other suitable output. Before an output is provided it can be beneficial to confirm that a prospective critical event that has been detected is a critical event. This can help to avoid alarms or other calls for assistance being raised unnecessarily.

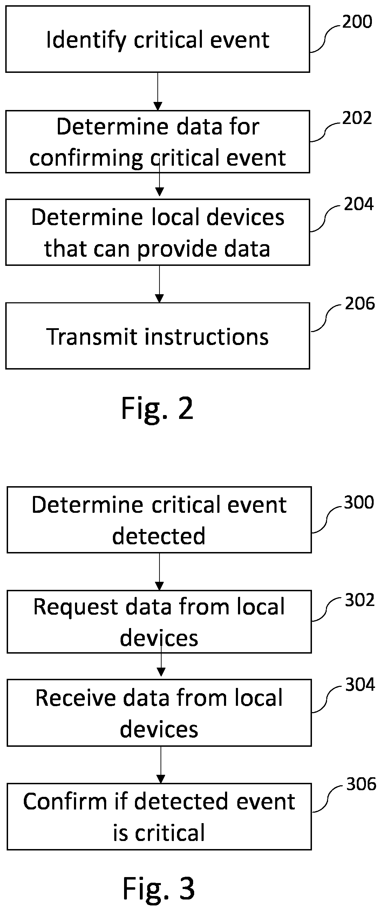

Fig. 2 shows an example method that can be used to set up a system 100 to enable confirmation of detection of a critical event. The method could be implemented in a system 100 as shown in Fig. 1 and/or in any other suitable type of system. The method of Fig. 2 can be used to set up a system 100 so that detection of critical events can be confirmed.8

The method of Fig. 2 can be performed by a coordinating device. The coordinating device could be a user electronic device within the system 100, a server that can be in communication with the devices 102 in the system 100 or any other suitable device. The coordinating device could enable the method of Fig. 2 to be performed during a set up phase of the system 100. The set up can be performed based on a user selection of critical events to be monitored and/or a selection of sensors to be used. For instance, if it is detected that a user has indicated that one or more events are to be classed as critical events then the set up can be performed. A user can use a smart phone application or any other means to indicate that the one or more events are to be classed as critical events.

The set up phase 100 of the system could be performed when the coordinating device detects that it is within a communication range of one or more other devices, in response to a user input, in response to a wearable device being activated, or could be performed at any other suitable time or in response to any other suitable trigger event.

At block 200 the method comprises identifying at least one event that is detectable by a first device as a critical event. The first device can be any suitable device in the system. In some examples the first device can be the coordinating device that performs the method of Fig. 2. In some examples the first device can be a different device to the coordinating device. In such examples the first device can be configured to communicate with an apparatus that can implement the method of Fig. 2.

The first device can be associated with a user 104. In some examples the first device could be a user electronic device such as a smart phone or tablet computer that is used by or belongs to a user 104. In some examples the first device could be a wearable device that is worn by the user.

The first device can comprise one or more sensors 106 that can be configured to detect the critical event. The events that can be detected by the first device are determined by the sensors 106 of the first device. That is, the sensors 106 that are available on the first device will define which events can be detected by the first device.

The sensors 106 available on the first device can be any suitable type of sensor. In some examples the sensors could comprise any one or more of a heart rate sensor, a photoplethysmography sensor, a temperature sensor, a motion sensor, a pressure sensor, an inertial measurement unit, a microphone, or any other suitable sensor or combination of sensors 106.

The sensors 106 that are available on the first device, and/or any other devices 102 in a system 100 can have multiple uses. For example, the sensors 106 do not need to be dedicated to health monitoring. An IMU could be used to monitor movement of a user for providing inputs to a device or for controlling a game or mediated reality. The information collected for such purposes could be reused by a coordinating device.

Identifying a critical event can comprise establishing which of the events that can be detected are classed as critical events. The identifying of a critical event distinguishes the critical event from other events that can be detected by the first device. Identifying a critical event causes the event to be classed in the subset of critical events. The apparatus can identify an event as critical by accessing a database of detectable events and determining if an event is indicated as critical in the database. An event can be indicated as critical in the database by being flagged appropriately.

At block 202 the method comprises determining data for confirming that a potential critical event detected by the first device is a critical event. The coordinating device can determine what additional data would be suitable for use to provide confirmation that a prospective critical event is a critical event.

Determining data for confirming that a potential critical event detected by the first device is a critical event can comprise accessing metadata associated with the critical event. The metadata can be stored in a database and associated with the type of critical event or can be stored in any other suitable location. In some examples a task based process could be used and in such examples the metadata could be part of the parameters of a task.

The data that can be used to confirm that a potential critical event detected by the first device is a critical event can be data obtained from the same type of sensor 106 or a similar type of sensor 106 as used by first device. For instance, both the first device and one or more of the other local devices could comprise heart rate sensors.

In some examples the data that can be used to confirm that a potential critical event detected by the first device is a critical event can be data obtained from a different type to the sensor used by first device. For example, the first device could comprise a heart rate sensor and the other device could comprise other types of sensors such as pressure devices or IMUs.

The data that can be used to confirm that a potential critical event detected by the first device is a critical event can be data obtained from multiple sensors. The number of sensors that are used to provide the data can be dependent upon the required accuracy for detection, the number of sensors available, the types of sensors available and/or any other suitable factor.

At block 204 the method comprises determining one or more local devices 102 capable of providing the data. The local devices can comprise other devices within a system 100 as shown in Fig. 1. The local devices can comprise a user electronic device, wearable device or any other suitable type of device.

The apparatus can determine which local devices 102 are in the area of the coordinating device and with which the coordinating device can communicate. The apparatus can also determine the types of data that can be collected by the respective devices that it can communicate with. For example, it can determine the types of sensors 106 that are available and/or the configuration of the sensors 106. The apparatus can determine whether the data collected by these sensors 106 would be suitable for confirming if an event is a critical event. For example, it can determine if the data collected by the sensors 106 is included within the data that is determined at block 202.

At block 206 the apparatus transmits instructions to the one or more local devices. The instructions can be transmitted to the local devices that have been determined to be capable of providing the data. The instructions that are transmitted indicate the data that is to be recorded by the respective local devices. The instructions can indicate the type of data that is to be recorded, the time interval for which the data is to be recorded, and/or any other suitable instructions. If a device comprises multiple types of sensors, or if the sensors are capable of obtaining multiple types of data then the instructions can be provided for respective types of data and/or sensors.

In some examples the instructions can indicate the purpose of the requested data. That is there can be an indication of what type of event the apparatus is trying to confirm with the requested data. In some examples the instructions might not indicate the purpose of the requested data. In such cases a local device need not be aware of the purpose of the collected data but can just collect and transmit it as instructed.

The instructions can be transmitted at any suitable time. The instructions can be transmitted during a set up procedure of at least one of the first device and the one or more local devices. The set up procedure can occur when at least one of the first device and the one or more local devices is turned on or if a health monitoring application is activated, when the first devices and one or more of the local devices are moved relative to each other so that they can communicate with each other, or following any other suitable trigger event.

Fig. 3 shows another example method. The method could be implemented in a system 100 as shown in Fig. 1 and/or in any other suitable type of system. The method or Fig. 3 can occur after the method of Fig. 2 has been performed. The method of Fig. 3 can be used to confirm that critical event has occurred.

The method of Fig. 3 can be performed by a coordinating device. The method of Fig. 3 can be performed by the same device that performs the method of Fig. 2. The coordinating device could be a user electronic device, a server that can be in communication with the devices 102 in the system 100 or any other suitable device. The coordinating device could enable the method of Fig. 3 to be performed during an event detection phase. The event detection phase could be performed whenever the outputs of one or more sensors 106 of the first device have a sufficient correlation with an expected output for a critical event.

At block 300 the method comprises determining that a prospective critical event has been detected. In some examples the prospective critical event can be detected by the apparatus or device that performs the method of Fig. 3. In such examples the apparatus would receive an output from one or more sensors and use that output to determine that a prospective critical event has been detected.

The sensors that are used to detect the prospective critical event can be part of the device that performs the method of Fig. 3 or can be part of any other device in a system 100. For instance, a different device could collect sensor data and send the sensor data to the device. The device could then use the received sensor data to determine that a prospective critical event has occurred.

In other examples the prospective critical event could be detected by a different apparatus or device to the device or apparatus that performs the method of Fig. 3. In such cases the apparatus or device that detects the prospective critical event sends a notification to the apparatus or device that implements the method of Fig. 3. The notification can indicate that the prospective critical event has been detected by at least one of the one or more local devices.

A prospective critical event can be detected if the outputs of one or more sensors 106 the first device have a sufficient correlation with an expected output for a critical event. For example, if a heart rate monitor and/or an IMU provides an output that has a high correlation with the expected output for a critical event.

The level of correlation that is needed between the detected outputs and the expected outputs to cause an output to be determined to indicate a prospective critical event can be determined by task parameters or by any other suitable means. The level of correlation that is needed to cause an output to be determined to indicate a prospective critical event can be dependent upon the type of event, the type of sensors that are used to obtain the outputs, and/or any other suitable factors.

At block 302 the method comprises requesting data from one or more local devices. The data that is requested is collected by one or more local devices in accordance with instructions previously provided by the apparatus. The instructions can be provided during a set up phase as shown in Fig. 2 or during any other suitable process.

The requests can be sent using any suitable communication protocol. In some examples the requests can be sent to all of the local devices. For instance, the apparatus can broadcast a request for data. In other examples the requests can be sent to one or more specific local devices. For example, the request could be sent only to the local devices that have been sent instructions.

In some examples the request can specify that data that is being requested. In some examples the request can specify the time interval for which the data is requested.

The request for data can be transmitted automatically. That is the request can be sent without any user input or other trigger condition being satisfied.

At block 304 the apparatus receives the data from the one or more local devices. The data can be received using any suitable communication means.

At block 306 the apparatus uses the received data to confirm if the prospective critical event is a critical event. Using the received data to confirm if the prospective critical event is a critical event can comprise analyzing a correlation between the received data and expected data for the critical event.

If there is a sufficient correlation between the received data and the expected data then the critical event can be confirmed. If there is not sufficient correlation between the received data and the expected data then the critical even is not confirmed.

An output can be provided by one or more devices 102 within the system 100 based on whether a critical event is confirmed. If a critical event is confirmed then the outputs could comprise an instruction to one or more local devices to provide an alert, a communication to an assistance provider, or any other suitable output. If a critical event is not confirmed then different outputs could be provided. In some examples there might be no outputs if no critical event is confirmed. In other examples a user could be notified that a critical event was suspected but not confirmed. This could alert the user to check that their devices 102 are being used correctly, to make a non-urgent request for health care or to perform any other suitable action.

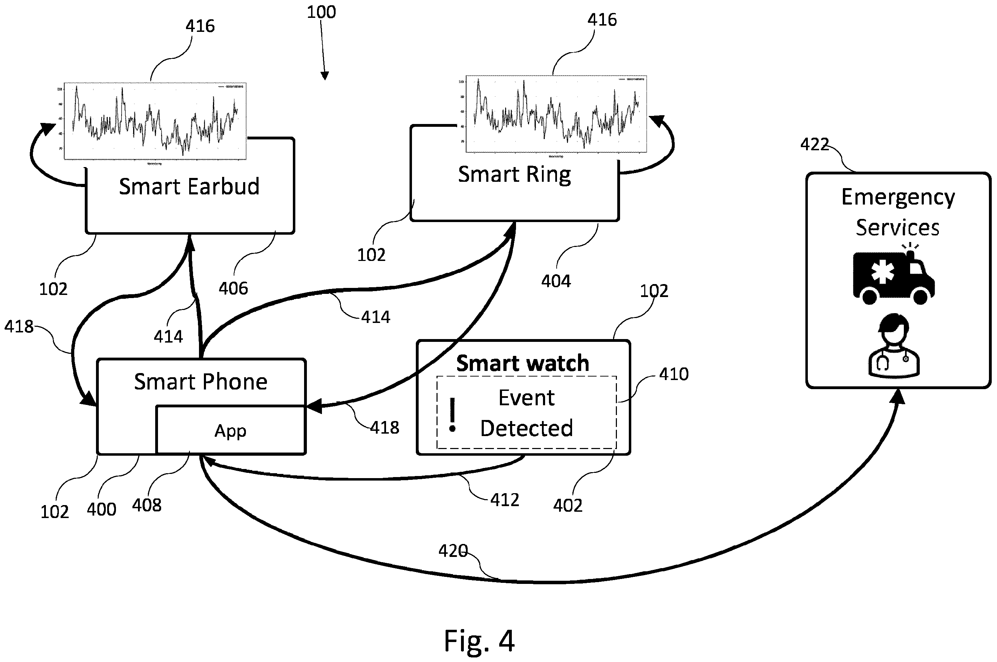

Fig. 4 shows an example control flow that could be used in some examples of the disclosure. In this example the system 100 comprises four devices 102. The devices 102 comprise a smart phone 400 and multiple wearable devices. The multiple wearable devices comprise a smart watch 402, a smart ring 404, and a smart earbud 406. The wearable devices are all worn by the same user on different body parts. The wearable devices are all worn by the user of the smart phone 400. Other types and arrangements of devices 102 can be used in other examples.

The respective devices 400-406 can all comprise one or more sensors 106 that can be used to detect a critical event. For example, the respective devices 102 can comprise one or more of a heart rate sensor, a photoplethysmography sensor, a temperature sensor, a motion sensor, a pressure sensor, an inertial measurement unit, a microphone, or any other suitable sensor.

The smart phone 400 can comprise an application 408. The application 408 can be stored in a memory of the smart phone 400 and/or can be otherwise accessed by the smartphone 400. The application 408 can enable communication between the smart phone 400 and the wearable devices. The application 408 can be companion application for the wearable devices. The application 408 can enable control of one or more of the wearable devices. The application 408 can enable an exchange of data between the smart phone 400 and the respective wearable devices. In the example of Fig. 4 one application 408 is shown in the smart phone 400. In this case the same application 408 can be used to communicate with multiple wearable devices. In some examples different applications 408 can be used to communicate with different wearable devices.

In this example the smart phone 400 acts as a coordinating device for the system 100. In this example the smart phone 400 can perform the methods shown in Fig. 2. This method can be performed at a set up phase. The set up phase can be performed when a user 104 uses one or more of the devices for a first time or starts to use one or more of the wearable devices or after any other suitable trigger event.

During the set up phase one or more critical events that can be detected by the smart phone and/or any of the wearable devices in the system are identified. The data that can be used to confirm the critical event can also be identified. This can be data that can be used to increase the certainty of the determination of a critical event.

The smartphone 400 can determine which of the wearable devices can be used to collect such data. The smartphone 400 can then send appropriate instructions to the respective devices. The instructions can indicate the data that is to be recorded, the time interval for which the data is to be collected, and/or any other suitable instructions.

The wearable devices can collect and store data in accordance with the instructions received from the smart phone 400.

In the control flow of Fig. 4 at block 410 the smart watch 402 detects a prospective critical event. In this example the prospective critical event is a fall. Other types of critical events could be detected in other implementations.

The fall can be detected by an IMU or other suitable sensors 106 on the smart watch 402. A prospective fall is determined to be detected if the output of the IMU, or other sensor, sufficiently correlates with the expected output for a fall.

At block 412 the smart watch 402 sends data to the smart phone 400. The data can be collected by one or more sensors from the smart watch 402.

The smart phone 400 receives the data 412. The smart phone 400 can process the received data to determine whether the event detected by the smart watch 402 is a prospective critical event.

The smart phone 400 can perform any suitable process to confirm whether the received data corresponds to a prospective critical event. If a prospective critical event is detected then the smart phone 400 can implement methods and processes to confirm whether the prospective critical event is a critical event. In examples of the disclosure the smart phone 400 can perform the methods of Fig. 3 or could use any other suitable method.

In the example of Fig. 4, to confirm that the prospective critical event is a critical event the smart phone 400 requests data from the other wearable devices at block 414. The smart phone 400 can request data from multiple other devices. The smart phone 400 can also obtain data from one or more sensors in the smart phone 400. In this case the smart phone 400 requests data from the smart ring 404 and the smart ear bud 406. The requests 414 to the smart ring 404 and the smart ear bud 406 can be sent sequentially or at the same time.

In this case the prospective critical event is a fall. The fall could be detected by IMUs or other suitable types of sensors. In this example both the smart ring 404 and the smart ear bud 406 can comprise IMUs. Therefore, both the smart ring 404 and the smart ear bud 406 have previously received instructions to record data that can be used to confirm a fall. The requests 414 can therefore be sent to both the smart ring 404 and the smart ear bud 406.

The spart ring 404 and/or the smart ear bud 406 could also comprise other types of sensors such as photoplethysmography sensors or any other suitable types of sensors. In such cases the smart ring 404 and/or the smart ear bud 406 have previously received instructions to record data from such sensors. This data can be requested by the smart phone 400 as appropriate. For example, the data from photoplethysmography sensors could be used to monitor heart rates, or other biological parameters which might be affected by a fall.

As the smart ring 404 and the smart ear bud 406 have both previously received instructions to record data that can be used to confirm a fall they have collected data 416 and stored the data 416 within appropriate memories. The data 416 can comprise the outputs from one or more sensors 106, data obtained by processing outputs from one or more sensors 106, and/or any other suitable data.

In response to the requests 414 from the smart phone 400 the smart ring 404 and the smart ear bud 406 will retrieve the appropriate data 416 and, at block 418, send the data 416 to the smart phone 400.

The data 416 is received by the smart phone 400 and used by the smart phone 400 to confirm if the prospective critical event that was detected at block 410 is a real critical event. In some examples the application 408 can process the data 416 from the smart ring 404 and the smart ear bud 406 to confirm the critical event.

Any suitable process or procedure can be implemented to use the data 416 to confirm the critical event. In some examples the process or procedure can comprise a clustering algorithm. The clustering algorithm can be implemented using machine learning or any other suitable means. The clustering algorithm can be configured to determine a similarity between the received data 416 and the data collected by the smart watch 410 that originally detected the prospective critical event. In some examples the clustering algorithm can be configured to determine a similarity between the received data 416 and expected data for a critical event.

The data 416 can be considered to confirm the critical event if there is a sufficient correlation between the received data 416 and the expected outputs or other detected outputs. For example, if the distance (such as the Euclidean distance) between a parameterized expected output for a critical event and the decision outcome of processing the received data 416 is sufficiently close to the mean then the critical event is confirmed.

If a critical event is confirmed then, at block 420, the smart phone 400 provides an output. The output that is provided can be dependent upon the type of critical event that is detected. In this example the detected critical event is a fall and the output 420 comprises contacting emergency services 422 or other assistance provider. Different outputs can be provided for different critical events.

If a critical event is not confirmed then the smart phone 400 might not provide any output. In some examples a different output could be provided such as a notification or a record of a suspected critical event.

In the example of Fig. 4 the smart phone 400 requests data from other devices 102 to confirm if the detected event is a critical event. In this case the smart phone 400 requests data from the smart ring 404 and the smart earbud 406. In other examples the smart phone 400 might not need to request the data from any other devices 102. For instance, the smart phone 400 could just request additional data from the smart watch 402.

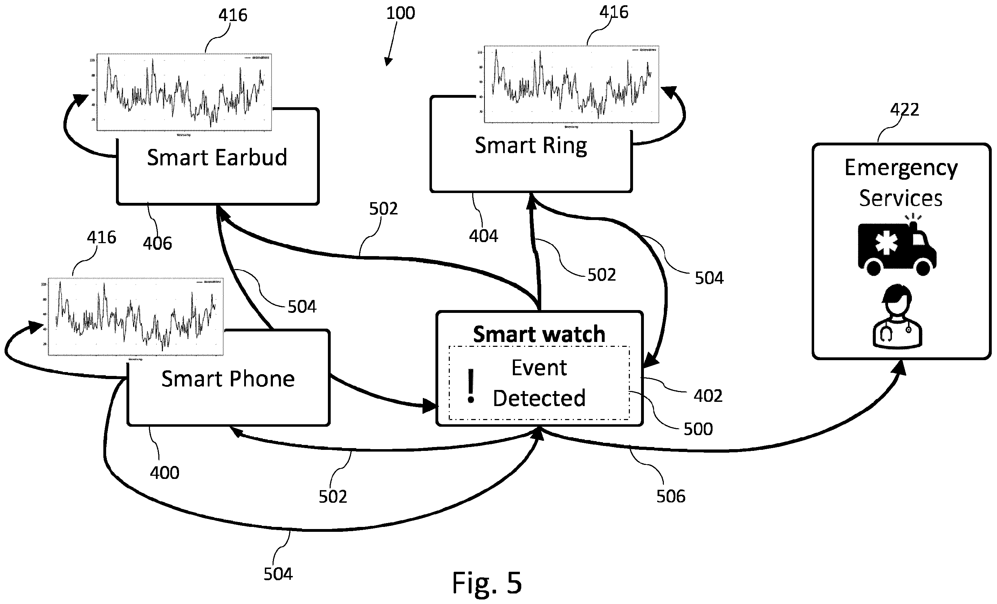

Fig. 5 shows another example control flow that could be used in some examples of the disclosure. In this example the system 100 comprises a smart phone 400 and multiple wearable devices comprising a smart watch 402, a smart ring 404, and a smart earbud 406. The smart phone 400 and the multiple wearable devices can be as shown in Fig. 4. In the example of Fig. 5 the smart phone 400 and the multiple wearable devices are configured to communicate in a peer to peer to arrangement.

Fig. 5 shows an example of an event detection. Prior to the event detection the system 100 can be configured to record data that can be used for confirmation of critical events. The methods of Fig. 2, or any other suitable methods can be used to configure the system 100 to record data that can be used for confirmation of critical events.

In this example the smart phone 400 and the smart ring 404 and the smart earbud 406 can all be instructed to record data that can be used to confirm a prospective critical event detected by the smart watch 402.

In the control flow of Fig. 5 at block 500 the smart watch 402 detects a prospective critical event. In this example the prospective critical event is a fall. Other types of critical events could be detected in other implementations.

The fall can be detected by an IMU or other suitable sensors 106 on the smart watch 402. A prospective fall is determined to be detected if the output of the IMU, or other sensors, sufficiently correlates with the expected output for a fall. For example, sensors that monitor physiological parameters such as heart rate could be expected to show a sudden peak if a fall occurs.

At block 502 the smart watch 402 sends a notification to other local devices requesting data that can be used to confirm whether the prospective critical event is a critical event. In this case the other local devices comprise the smart phone 400, the smart ring 404 and the smart earbud 406. Other local devices could be used in other examples. The requests 502 to the smart phone 400, the smart ring 404 and the smart ear bud 406 can be sent sequentially or at the same time.

In this case the prospective critical event is a fall. The fall could be detected by IMUs or other suitable types of sensors. In this example the smart phone 400, the smart ring 404 and the smart ear bud 406 can comprise IMUs. Therefore, the smart phone 400, the smart ring 404 and the smart ear bud 406 have previously received instructions to record data 416 from the IMUS that can be used to confirm a fall. The recorded data 416 can comprise the outputs from one or more sensors 106, data obtained by processing outputs from one or more sensors 106, and/or any other suitable data.

In response to the requests 502 from the smart watch 402, the smart phone 400, the smart ring 404 and the smart ear bud 406 will retrieve the appropriate data 416 and, at block 504, send the data 416 to the smart watch 402.

The data 416 is received by the smart watch 402 and used by the smart watch 402 to confirm if the prospective critical event that was detected at block 500 is a real critical event.

Any suitable process or procedure can be implemented to use the data 416 to confirm the critical event. For example, the smart watch 402 can implement clustering algorithms or any other suitable processes or procedures.

If a critical event is confirmed then, at block 506, the smart watch 402 provides an output 506. The output 506 that is provided can be dependent upon the type of critical event that is detected. In this example the detected critical event is a fall and the output 506 comprises contacting emergency services 422 or other assistance provider. Different outputs can be provided for different critical events.

If a critical event is not confirmed then the smart watch 402 might not provide any output. In some examples a different output could be provided such as a notification or a record of a suspected critical event.

In the example of Figs. 4 and 5 the data used to confirm if a critical event has occurred are all obtained from the same or similar types of sensors. In this case multiple IMUs can be worn on different parts of the user 104. The respective IMUs can all detect the motion of the user 104 as they fall. The outputs of the respective IMUs can be collected and processed by a coordinating device such as the smart phone 400. In other examples different types of sensors 106 can be used. This could enable different types of data to be collected. For instance, an IMU on a wearable device could detect the motion of a user while a microphone on another device could detect the sound of the fall.

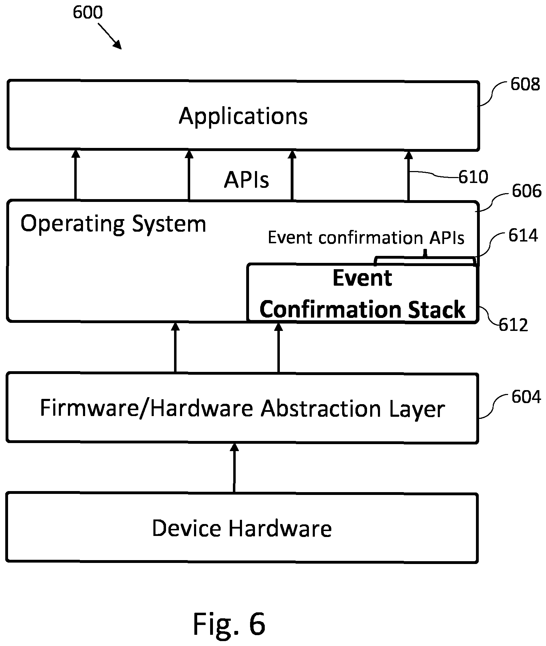

Fig. 6 shows an example software architecture 600 that can be used in examples of the disclosure. The software architecture 600 comprises device hardware 602, firmware/hardware abstraction layer 604, an operations system 606, and one or more applications 608. One or more application programming interfaces (APIs) are provided between the operating system 606 and the applications 608. The software architecture can be provided in any suitable devices 102.

In examples of the disclosure the event confirmation stack 612 can be provided as part of the operating system 606. Event confirmation APIs 614 can be provided between the event confirmation stack and the appropriate applications. Providing the event confirmation stack 612 as part of the operating system can make the examples of disclosure simple to implement.

The event confirmation stack 612 can be enabled in the operating system 606 so as to enable a device 102 to automatically trigger the examples of the disclosure and obtain the appropriate data for confirming a critical event.

The other devices 102 in the system 100 that provide the requested data do not need to have a compatible operating system. That is different devices 102 with a system can have different operating systems. This can enable the systems 100 to comprise any suitable devices 102 that can collect the appropriate data. The devices could comprise internet of things devices. The internet of things devices could be passive or ambient devices.

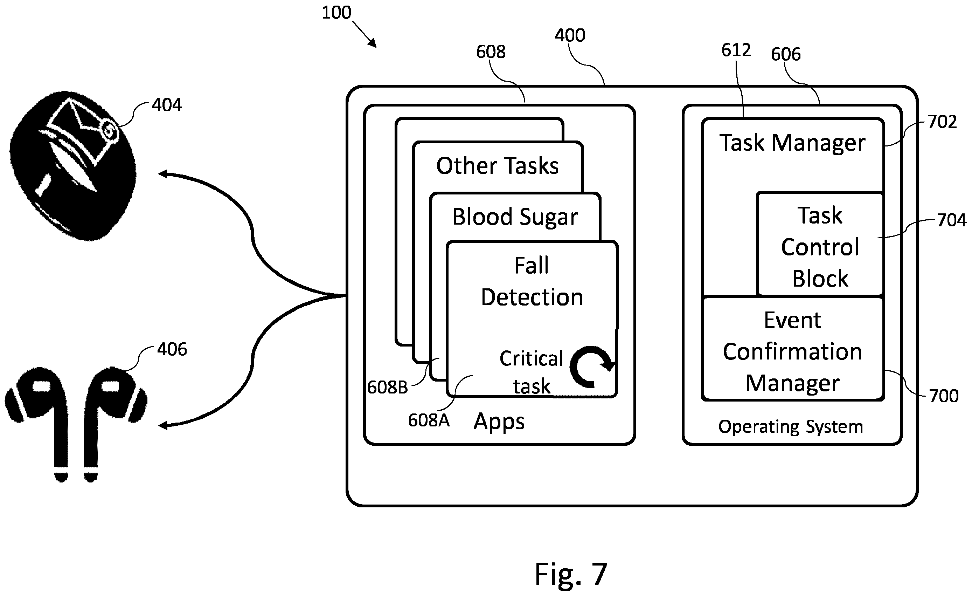

Fig. 7 shows another example system 100 that can be used in examples of the disclosure. This system comprises a smart phone 400, a smart ring 404 and smart earbuds 406. Other types of devices or combinations of devices could be used in other examples.

The smart phone 400 can comprise a software architecture 600. The software architecture 600 can be as shown in Fig. 6. Only the applications 608 and the operating system 606 are shown in Fig. 7.

In the example of Fig. 7 the operating system 612 comprises the event confirmation stack 612. The event confirmation stack 612 can be configured to implement methods such as the methods of Fig. 2 and Fig. 3.

The event confirmation stack 612 comprises an event confirmation manager 700, a task manager 702 and a task control block 704.

The task control block 704 can store information relating to tasks that can be performed or implemented by the smart phone 400. The information can comprise an indication of whether the task relates to a critical event. For instance, certain events can be flagged as critical. The events can be flagged as critical during the development of the event confirmation stack 612 or at any other suitable time. The task control block 704 can also store information such as metadata or other parameters relating to the critical events. The metadata or other parameters can comprise information such as, the types of sensors 106 or devices 102 that can be used to collect data that can confirm if a critical event has occurred, the preferred data that can be used to confirm if a critical event has occurred, the expected data outputs for certain types of sensors 106 and/or devices 102, a required correlation between recorded data and expected data needed to confirm events, or any other suitable data.

The task manager 702 can use information from the task control block 704 when implementing tasks. For example, it can use information from the task control block 704 to identify a task as relating to a critical event and implementing an appropriate confirmation procedure.

If a task has been identified as relating to a critical even then the event confirmation manager can implement a confirmation procedure, such as the method of Fig. 3, as appropriate.

In the example of Fig. 7 multiple applications 608 are shown. A first application 608A is used for fall detection and a second application 608Bis used for monitoring blood sugar levels. Other applications can be used in other examples.

In this case the fall detection is flagged as a critical task because it relates to detecting a critical event. The task manager 702 can identify that the fall detection application 608A relates to a critical event and can flag the application 608A as appropriate. When the fall detection application 608A is being set up the set up procedure of Fig. 2 or any other suitable methods can be implemented. Such methods can be implemented automatically without any specific user input. The methods enable the data that is to be used for confirmation of a critical event to be recorded by devices 102 such as the smart ring 404 and the smart earbud 406.

If an event is detected by the fall detection application 608A then the methods of Fig. 3, or other suitable methods, can be implemented automatically so as to enable confirmation of whether a critical event has occurred. The metadata or parameters stored by the task control block 704 can be used to confirm if the critical event has occurred.

Examples of the disclosure provide a method where an event detected by a single device 102 can be confirmed with data obtained from one or more devices 102. The confirmation can happen through the coordination of the respective device 102. This can enable the confirmation to happen automatically without any input from the user 104 of any of the devices 102. The confirmation of the outputs of one device 102 by data recorded by one or more other devices can provide reliability of the detection of critical events. This can enable consumer-grade devices provide a reliability comparable to that of medical grade devices.

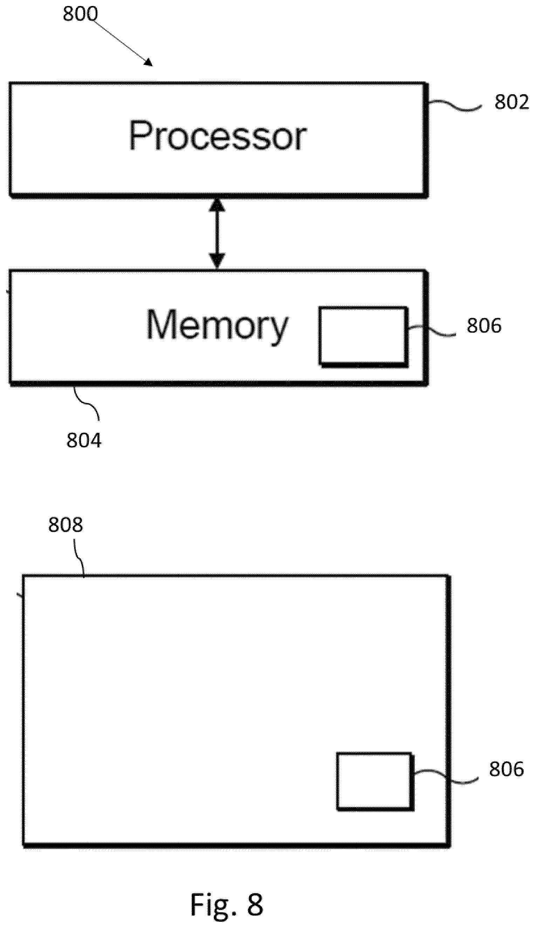

Fig. 8 shows an example controller 800 that can be used in some examples of the disclosure. The controller can 800 can be provided within devices 102 as shown in the Figs. The controller 800 can provide apparatus for implementing examples of the disclosure.

Implementation of the controller 800 may be as controller circuitry. The controller 800 may be implemented in hardware alone, have certain aspects in software including firmware alone or can be a combination of hardware and software (including firmware).

As illustrated in Fig. 8 the controller 800 can be implemented using instructions that enable hardware functionality, for example, by using executable instructions 806 in a general-purpose or special-purpose processor 802 that may be stored on a machine readable storage medium (disk, memory etc.) to be executed by such a processor 802.

The processor 802 is configured to read from and write to the memory 804. The processor 802 may also comprise an output interface via which data and/or commands are output by the processor 802 and an input interface via which data and/or commands are input to the processor 802.

The memory 804 stores instructions 806, program or code that controls the operation of an apparatus when loaded into the processor 802. The instructions 806, program or code, provide the logic and routines that enable the apparatus to perform the methods illustrated in the accompanying Figs. The processor 802 by reading the memory 804 is able to load and execute the instructions 806, program or code.

The apparatus comprises:
at least one processor 802; and
at least one memory 804 storing instructions 806 that, when executed by the at least one processor 802, cause the apparatus at least to:
   determining 300 that a prospective critical event has been detected;
   requesting 302 data from one or more local devices 102 wherein the data is collected in accordance with instructions previously provided by the apparatus;
   receiving 304 the data from the one or more local devices; and
   using 306 the received data to confirm if the prospective critical event is a critical event.

The apparatus comprises:
at least one processor 802; and
at least one memory 804 storing instructions 806 that, when executed by the at least one processor 802, cause the apparatus at least to:
   identifying 200 at least one event that is detectable by a first device as a critical event;
   determining 202 data for confirming that a potential critical event detected by the first device is a critical event;
   determining 204 one or more local devices capable of providing the data; and
   transmitting 206 instructions to the one or more local devices wherein the instructions indicate the data that is to be recorded by the respective local devices.

As illustrated in Fig. 8 the instructions 806, program or code can arrive at the apparatus via any suitable delivery mechanism 808. The delivery mechanism 808 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid-state memory, an article of manufacture that comprises or tangibly embodies the computer program, the instructions 806, program or code. The delivery mechanism may be a signal configured to reliably transfer the computer program the instructions 806, program or code. The apparatus may propagate or transmit the computer program the instructions 806, program or code as a computer data signal.

The term "non-transitory," as used herein, is a limitation of the medium itself (that is, tangible, not a signal) as opposed to a limitation on data storage persistency (that is, RAM vs. ROM).

Computer program instructions for causing an apparatus to perform at least the following or for performing at least the following:
determining 300 that a prospective critical event has been detected;
requesting 302 data from one or more local devices 102 wherein the data is collected in accordance with instructions previously provided by the apparatus;
receiving 304 the data from the one or more local devices; and
using 306 the received data to confirm if the prospective critical event is a critical event.

Computer program instructions for causing an apparatus to perform at least the following or for performing at least the following:
identifying 200 at least one event that is detectable by a first device as a critical event;
determining 202 data for confirming that a potential critical event detected by the first device is a critical event;
determining 204 one or more local devices capable of providing the data; and
transmitting 206 instructions to the one or more local devices wherein the instructions indicate the data that is to be recorded by the respective local devices.

The computer program instructions may be comprised in a computer program, a non-transitory computer readable medium, a computer program product, a machine readable medium. In some but not necessarily all examples, the computer program instructions may be distributed over more than one computer program.

Although the memory 804 is illustrated as a single component/circuitry it may be implemented as one or more separate components/circuitry some or all of which may be integrated/removable and/or may provide permanent/semi-permanent/ dynamic/cached storage.

Although the processor 802 is illustrated as a single component/circuitry it may be implemented as one or more separate components/circuitry some or all of which may be integrated/removable. The processor 802 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory or memories that work together to cause an apparatus, such as a mobile phone or server, to perform various functions and
(c) hardware circuit(s) and or processor(s), such as a microprocessor(s) or a portion of a microprocessor(s), that requires software (for example, firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in the accompanying Figs may represent steps in a method and/or sections of code in the computer program or instruction 106. The illustration of a particular order to the blocks does not necessarily imply that there is a required or preferred order for the blocks and the order and arrangement of the block may be varied. Furthermore, it may be possible for some blocks to be omitted.

Where a structural feature has been described, it may be replaced by means for performing one or more of the functions of the structural feature whether that function or those functions are explicitly or implicitly described.

The apparatus can be provided in an electronic device, for example, a mobile terminal, according to an example of the present disclosure. It should be understood, however, that a mobile terminal is merely illustrative of an electronic device that would benefit from examples of implementations of the present disclosure and, therefore, should not be taken to limit the scope of the present disclosure to the same. While in certain implementation examples, the apparatus can be provided in a mobile terminal, other types of electronic devices, such as, but not limited to: mobile communication devices, hand portable electronic devices, wearable computing devices, portable digital assistants (PDAs), pagers, mobile computers, desktop computers, televisions, gaming devices, laptop computers, cameras, video recorders, GPS devices and other types of electronic systems, can readily employ examples of the present disclosure. Furthermore, devices can readily employ examples of the present disclosure regardless of their intent to provide mobility.

The apparatus can be provided in an electronic device, for example, a mobile terminal, according to an example of the present disclosure. It should be understood, however, that a mobile terminal is merely illustrative of an electronic device that would benefit from examples of implementations of the present disclosure and, therefore, should not be taken to limit the scope of the present disclosure to the same. While in certain implementation examples, the apparatus can be provided in a mobile terminal, other types of electronic devices, such as, but not limited to: mobile communication devices, hand portable electronic devices, wearable computing devices, portable digital assistants (PDAs), pagers, mobile computers, desktop computers, televisions, gaming devices, laptop computers, cameras, video recorders, GPS devices and other types of electronic systems, can readily employ examples of the present disclosure. Furthermore, devices can readily employ examples of the present disclosure regardless of their intent to provide mobility.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to 'comprising only one...' or by using 'consisting.'

In this description, the wording 'connect', 'couple' and 'communication' and their derivatives mean operationally connected/coupled/in communication. It should be appreciated that any number or combination of intervening components can exist (including no intervening components), i.e., to provide direct or indirect connection/coupling/communication. Any such intervening components can include hardware and/or software components.

As used herein, the term "determine/determining" (and grammatical variants thereof) can include, not least: calculating, computing, processing, deriving, measuring, investigating, identifying, looking up (for example, looking up in a table, a database, or another data structure), ascertaining and the like. Also, "determining" can include receiving (for example, receiving information), accessing (for example, accessing data in a memory), obtaining and the like. Also, " determine/determining" can include resolving, selecting, choosing, establishing, and the like.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can', or 'may' refers to a particular instance in a class of examples. A property of the instance can be a property of only that instance or a property of the class or a property of a sub-class of the class that includes some but not all the instances in the class. It is therefore implicitly disclosed that a feature described with reference to one example but not with reference to another example, can where possible be used in that other example as part of a working combination but does not necessarily have to be used in that other example.

As used herein, "at least one of the following: " and "at least one of " and similar wording, where the list of two or more elements are joined by "and" or "or" mean at least any one of the elements, or at least any two or more of the elements, or at least all the elements.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a', 'an' or 'the' is used in this document with an inclusive not an exclusive meaning. That is any reference to X comprising a/an/the Y indicates that X may comprise only one Y or may comprise more than one Y unless the context clearly indicates the contrary. If it is intended to use 'a', 'an' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

The above description describes some examples of the present disclosure however those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description of the examples of the present disclosure.

Whilst endeavoring in the foregoing specification to draw attention to those features believed to be of importance the Applicant may seek protection via the claims in respect of any patentable feature or combination of features hereinbefore referred to and/or shown in the drawings whether or not emphasis has been placed thereon.

## Claims

1. An apparatus comprising means for:
determining that a prospective critical event has been detected;
requesting data from one or more local devices wherein the data is collected in accordance with instructions previously provided by the apparatus;
receiving the data from the one or more local devices; and
using the received data to confirm if the prospective critical event is a critical event.

2. An apparatus as claimed in claim 1 wherein the means are for detecting the prospective critical event.

3. An apparatus as claimed in any preceding claim wherein the means are for receiving a notification that the prospective critical event has been detected by at least one of the one or more local devices.

4. An apparatus as claimed in any preceding claim wherein using the received data to confirm if the prospective critical event is a critical event comprises analyzing a correlation between the received data and expected data for the critical event.

5. A method comprising:
determining that a prospective critical event has been detected;
requesting data from one or more local devices wherein the data is collected in accordance with instructions previously provided by the apparatus;
receiving the data from the one or more local devices; and
using the received data to confirm if the prospective critical event is a critical event.

6. A computer program comprising instructions which, when executed by an apparatus, cause the apparatus to perform at least:
determining that a prospective critical event has been detected;
requesting data from one or more local devices wherein the data is collected in accordance with instructions previously provided by the apparatus;
receiving the data from the one or more local devices; and
using the received data to confirm if the prospective critical event is a critical event.

7. An apparatus comprising means for:
identifying at least one event that is detectable by a first device as a critical event;
determining data for confirming that a potential critical event detected by the first device is a critical event;
determining one or more local devices capable of providing the data; and
transmitting instructions to the one or more local devices wherein the instructions indicate the data that is to be recorded by the respective local devices.

8. An apparatus as claimed in claim 7 wherein the events that are detectable by the first device are determined, at least in part, by one or more sensors of the first device.

9. An apparatus as claimed in any of claims 7 to 8 wherein the first device comprises at least one of:
a heart rate sensor;
a photoplethysmography sensor;
a temperature sensor;
a motion sensor;
a pressure sensor;
an inertial measurement unit; or
a microphone.

10. An apparatus as claimed in any of claims 7 to 9 wherein identifying a critical event comprises accessing a database of detectable events and determining if an event is indicated as critical in the database.

11. An apparatus as claimed in any of claims 7 to 10 wherein determining data for confirming that a potential critical event detected by the first device is a critical event comprises accessing metadata associated with the critical event.

12. An apparatus as claimed in any of claims 7 to 11 wherein the instructions transmitted to the respective local devices comprises at least one of:
a type of data that is to be recorded; or
a time interval for which the data is to be recorded.

13. An apparatus as claimed in any of claims 7 to 12 wherein the instructions are transmitted during a set up procedure of at least one of the first device and the one or more local devices.

14. An apparatus as claimed in any of claims 7 to 13 wherein, at least one of the first device and the one or more local devices is a wearable device.

15. A method comprising:
identifying at least one event that is detectable by a first device as a critical event;
determining data for confirming that a potential critical event detected by the first device is a critical event;
determining one or more local devices capable of providing the data; and
transmitting instructions to the one or more local devices wherein the instructions indicate the data that is to be recorded by the respective local devices.

16. A computer program comprising instructions which, when executed by an apparatus, cause the apparatus to perform at least:
identifying at least one event that is detectable by a first device as a critical event;
determining data for confirming that a potential critical event detected by the first device is a critical event;
determining one or more local devices capable of providing the data; and
transmitting instructions to the one or more local devices wherein the instructions indicate the data that is to be recorded by the respective local devices.
